# EUROPEAN PATENT APPLICATION

(11) **EP 1 489 421 A1**
(43) Date of publication of application: **22.12.2004**
(21) Application number: 04290164.5
(22) Date of filing: 22.01.2004
(51) Int. Cl.: G01N 33/574

(54) **Dendritic cells for prognosis of metastatic evolution of tumors**

(30) Priority: 19.06.2003 EP 03291491
(71) Applicant: INSTITUT GUSTAVE ROUSSY, 94800 Villejuif (FR)
(72) Inventor: Angevin, Eric, 45700 Villevoques (FR); Spatz, Alain, 75016 Paris (FR)
(74) Representative: Vialle-Presles, Marie José

(57) **Abstract**

The invention relates to methods for prognostic of tumoral evolution and/or tumoral response to immunotherapy in a patient, by determining the number of dendritic cells in a sample taken from sentinel lymph nodes of said patient.

## Description

The invention relates to new methods for prognostic of tumoral evolution and/or tumoral response to immunotherapy.

There is now accumulating evidence that a wide range of human cancers elicit anti-tumor immune responses. Indeed, isolation of specific cytotoxic T lymphocytes (CTLs) has allowed to clone and characterize genes encoding human tumor-associated antigens (TAA) in different cancer types (BOON et al, Immunol.Today, 18, 267-268, 1997 ; ROSENBERG, Immunity, 10, 281-287, 1999). Melanoma is considered among the most immunogenic tumor models since numerous differentiation and shared tumor antigens have been identified and tumor-specific CTL responses contributing to spontaneous tumor immunosurveillance have been reported (MACKENSEN et al, J.Clin.Invest., 93, 1397-1402, 1994).

However, few patients receiving peptide-based vaccines so far developed significant specific immune responses in correlation to clinical regressions (COULIE et al, Curr.Opin.Immunol., 15, 131-137, 2003).

Mature dendritic cells (DCs) are the most potent antigen-presenting cells (APCs) capable of priming tumor-specific T cells (BANCHEREAU et al, Nature, 392, 245-252, 1998) and their use in cancer immunotherapy appears to be a promising way to elicit and expand efficient anti-tumor immune responses (NESTLE et al, Nat.Med. 4, 328-332, 1998; SCHULER-THURNER et al, J.Exp.Med., 195, 1279-1288, 2002). However, little is known about the role of DC in the natural immunosurveillance of cancer.

It is generally postulated that spontaneous immunity against tumor antigens are elicited by DCs maturing in response to various inflammatory signals, but the sites and conditions in which DCs capture and/or encounter tumor antigens remains largely unknown. Indeed, most studies addressed this question using non specific antibodies (LANA et al, Melanoma Res., 11, 401-410, 2001), while other reports describe the localization and phenotype of DCs in tumor samples without clinical correlates (BELL et al, J.Exp.Med., 190, 1417-1426, 1999).

Melanoma has become a paradigm for studying the interactions between tumor and host immune cells for the following reasons. First, the primary lesion occurs in the skin, the main residency compartment for immature Langerhans cells (LCs) of high migratory potential to skin-draining lymph nodes (LNs; BANCHEREAU et al, Annu.Rev.Immunol., 18, 767-811, 2000; GEISSMANN et al, J.Exp.Med., 196, 417-430, 2002). Second, sentinel LNs (SLNs) are privileged sites of both T cell priming and first metastases, the extent of SLN involvement representing an independent predictive factor for survival (COCHRAN et al, Ann.Surg.Oncol., 8, 13S-17S, 2001, STARZ et al, Cancer, 91, 2110-2121, 2001). Third, cutaneous and LN metastases appear to be the most sensitive tumor sites to vaccine-based immunotherapies (NESTLE et al, 1998, mentioned above; MARCHAND et al, Int.J.Cancer, 80, 219-230, 1999).]

Using a panel of antibodies recognizing DC subsets and maturation markers, the Inventors have addressed the clinical relevance of DC infiltration and maturation on regressing tumor lesions and sentinel SLNs in melanoma patients. They observed, in SLNs from a patient having spontaneous regression of metastatic melanoma, a dramatic infiltration of DCs expressing the DC-LAMP maturation marker (DC-Lamp⁺ DCs) in close contact with effector memory Melan-A/Mart-1 specific CTLs *in situ*.

DC-LAMP, also refered as CD208, is a lysosome-associated membrane glycoprotein, expressed in maturing DCs in the lysosomal major MHC class II compartment (DE SAINT-VIS et al., Immunity., 9, 325-336, 1998; US Patent 6,361,939). DC-Lamp precursor is a 416-amino acid protein, comprising a 381-amino acid extracellular domain, a hydrophobic transmembrane domain, and a 10-amino acid cytoplasmic domain containing a conserved GY lysosomal targeting motif, characteristic of LAMP family members.

Since SLNs are recognized as the first site of melanoma metastases, the Inventors postulated that the extent of DC-Lamp⁺ DC infiltrates may dictate the induction of efficient local anti-tumor immune responses. To further test his hypothesis, they analyzed a series of 19 metastatic SLNs, and found that a high number of DC-Lamp⁺ DCs in sentinel lymph nodes correlates with a tumor-free status after lymphadenectomy, and a favorable clinical outcome.

These findings of the Inventors demonstrate a pivotal role of DC-Lamp expressing DCs in melanoma immunosurveillance at the first sites of metastasis, and show that the extent of SLNs infiltration by DC-Lamp⁺ DCs is of prognostic value for evaluating the risk of metastasic dissemination of a tumor.

The extent of SLNs infiltration by DC-Lamp⁺ DCs is also of prognostic value for evaluating the clinical efficiency of anti-tumor immunotherapy. As demonstrated by the Inventors in the spontaneous regression case report described below, the dramatic infiltration of DC-Lamp⁺ DCs may be directly involved in the local expansion and differentiation of a protective tumor-specific immune response. Hence, the extent of infiltration by DC-Lamp⁺ DCs may allow to select patients with a pre-existing efficient immunosurveillance that could be successfully boosted by an anti-tumor immunotherapy. Anti-tumor immunotherapy includes in particular administration of tumor-specific vaccines containing tumor antigens delivered under various formulations (peptides, proteins, exosomes, nucleic acid sequences alone or in recombinant vectors, lysats or apoptotic bodies from tumor cells). Said tumor antigens can be delivered alone or in combination with adjuvants of immunity, such as oil emulsions, cytokines (IFNs, IL-2, GM-CSF,...), bacterial extracts (BCG, KompA/p40, LPS, ...) and more generally all ligands of the Toll-Like Receptors (ODN CpG sequences, poly-IC, poly-AU, flagellin,...), (ii) cytokines, such as IFNs or IL-2, (iii) adoptive transfer of lymphocytes.

The present invention thus provides a method for evaluating the risk of metastasic dissemination of a tumor in a patient wherein said method comprises determining the number of dendritic cells in a sample taken from sentinel lymph nodes of said patient.

The present invention also provides a method for evaluating the probability of efficiency of anti-tumoral immunotherapy in a patient wherein said method comprises determining the number of dendritic cells in a sample taken from sentinel lymph nodes of said patient.

According to a preferred embodiment of the methods of the invention said dendridic cells are DC-Lamp+ dendritic cells.

According to a preferred embodiment of the invention, said tumor is a melanoma.

Cell numbering techniques that are suitable for numbering dendritic cells, in particular DC-Lamp+ dendritic cells in a sample are well known in themselves. By way of exemple, one can selectively stain the DC-Lamp+ cells in a sample preparation and directly count them under microscope; alternatively, the cell numbering can be combined with the sorting of the DC-Lamp+ cells for instance by flow cytometry.

Typically, for selective staining and/or sorting of the DC-Lamp+ dendritic cells, one will use an anti-DC-Lamp polyclonal or monoclonal antibody, optionally conjugated with an appropriate label.

Antibodies that can be used to carry out the methods of the invention are known in themselves, or can easily be obtained by methods known in the art. By way of example of a commercially available anti-DC-Lamp antibody, one can mention the IgG1 antibody 104.G4, commercialized by BECKMAN COULTER, (Fullerton, USA). Other anti-DC-LAMP polyclonal or monoclonal antibodies may also be obtained by techniques familiar to those skilled in the art, as described for instance in US 6,361,939, or in the publication of DE SAINT-VIS et al., 1998,cited above.

Additionnally, other examples of known antibodies directed against dendritic cells could be used to characterize dendritic cell infiltrates such as anti-CD1a, anti-CD1c, anti-CD11c, anti-CD80, anti-CD83, anti-CD86, anti-CD123, anti-CD205 (anti-DEC-205), anti-CD207 (anti-Langerin/Lag), anti-CD209 (anti-DC-Sign), anti-S100 protein, anti-p55/fascin, anti-MHC class II, anti-CCR6, anti-CCR7, anti-BDCA-1, anti-BDCA-2, anti-BDCA-3, anti-BDCA-4, anti-ILT3, anti-ILT4.

The present invention will be further illustrated by the additional description which follows, which refers to non-limitative examples illustrating a correlation between the number of DC-Lamp⁺ DCs in sentinel lymph nodes and the clinical evolution of melanoma.

### EXAMPLES:

### MATERIALS AND METHODS

### Biological samples

All materials collected in this study were obtained after informed signed consent approved by the Institutional Review Board at the Gustave Roussy Institute and by the Legal Ethical Comittee.

### Case report.

The patient described in this study was a 28-year-old male with an atypical naevus syndrome who developed in December 2000 a right axillar lymphadenopathy corresponding to a melanoma metastasis. The patient reported in the previous months a pigmented nodular lesion in the right arm that had regressed spontaneously. This residual lesion was removed and a lymphadenectomy in the right axillar basin was performed. Histologically, the cutaneous lesion displayed typical features of regression associating the absence of melanoma cells, a flat epidermis and a fibrotic dermis containing ectasic vessels, heterogeneous melanin pigmentation and a dense lymphocytic infiltrate. The lymphadenectomy revealed 8 metastatic LNs out of 24. After a 10-months disease-free period, a right sub-clavicular mass developed that corresponded, at surgical examination, to a 2-cm diameter LN. Histologically, this LN was free of tumor cells and appeared almost completely necrotic with extensive peripheral fibrosis. LN samples were kept frozen for further analyses, while the primary cutaneous site was paraffin-embedded only. This patient remains disease-free 30 months after diagnosis without any adjuvant therapy.

### Series of 19 melanoma-positive SLN.

For this study, a consecutive series of 19 patients with a melanoma-positive SLN followed in our Institution between June 1999 and March 2002 were selected. All patients underwent surgical SLN resection at the time or a few weeks after excision of the primary cutaneous melanoma, according to the recommended procedures (COCHRAN et al, 2001, mentioned above). Few weeks after the SLN procedure, all underwent a regional completion lymphadenectomy in order to detect a metastatic extension in non-SLNs. The materials used consisted in paraffin-embedded sections from recorded paired SLNs and non-SLNs samples.

### Characterization of Melan-A/Mart-1-specific CTLs from peripheral blood. Immunophenotyping using PE-labeled HLA-A *0201/peptide tetramer complexes.

HLA-A*0201/Melan-A/Mart-1₂₆₋₃₅ A27L analog and control HLA-A*0201/HIVgag₇₇₋₈₅ were synthesized as described by MOTTA et al. (J. Immunol. 167:1795-1802, 2001). Total peripheral blood mononuclear cells (PBMCs) or immunopurified CD8⁺ T cells, obtained after magnetic cell sorting using a MiniMACS device (CD8⁺ T Cell Isolation Kit, Miltenyi Biotec, Bergisch Gladbach, Germany), were stained with the PE-labeled tetramers (5 nM) at room temperature for 45 min in PBS-2% BSA, washed once and stained for cell surface differentiation antigens using mAbs during 30 min at 4°C, washed again and analyzed by flow cytometry. Conjugated anti-CD3, -CD8, CD45RA mAbs were purchased from BD Biosciences (BD, Le Pont de Claix, France) and anti-TCRVβ2 and -TCRVβ3 were purchased from Beckman Coulter (Villepinte, France). Anti-CCR7 rat IgG2a mAb 3D12 (CCR for CC chemokine receptor; kindly provided by R. Forster, Erlangen, Germany) was incubated during 30 min at 4°C, washed and additionally stained with an APC-labeled goat anti-rat IgM+IgG (Southern Biotechnology, Birmingham, AL). Fluorescent isotype-matched mAbs were used as controls. Direct *ex-vivo* analysis was performed after acquisition of more than 10⁶ events on a FACSCalibur (BD) using the Cellquest software.

### Sorting, cloning, expansion and characterization of anti-Melan-A/Mart-1 CTLs.

CD3⁺CD8⁺HLA-A*0201/Melan-A/Mart-1₂₆₋₃₅⁺ were sorted by flow cytometry using FACS Vantage (BD) and cultured in V-bottomed 96 plates either in bulk or after direct cloning in limiting dilution on feeder cells consisting in irradiated allogeneic PBMCs, EBV-transformed LAZ509 B cells and MEL-FON tumor cells, a HLA-A2⁺ melanoma tumor cell line expressing the Melan-A/Mart-1 antigen (MOTTA et al, 2001, mentioned above). Growing clonal colonies were expanded at 37°C and 5% CO₂ in RPMI1640 medium (Gibco-BRL, Paisley, United Kingdom) supplemented with 10% human AB serum (Institut Jacques Boy, Reims, France), 50 IU/ml rHuIL-2 (Proleukin, Chiron, Rattingen, Gennany) and 3% T-cell growth factor, a supernatant of PHA/PMA allostimulated T cells. T cell clones were characterized for immunophenotype using HLA-A*0201/Melan-A/Mart-1₂₆₋₃₅ tetramers and mAbs directed against CD3, CD8 and for TCR-Vβ repertoire analysis with a panel of 21 anti-BV mAbs (IOTest Beta Mark, Beckman Coulter Immunotech, Marseille, France) and for cytotoxic activity in a standard 4h ⁵¹Cr-release assay against MEL-FON tumor cells and TAP-deficient EBV-transformed T2 cells loaded with 10 µg/ml of Melan-A/Mart-1₂₆₋₃₅ synthetic peptide. INFγ secretion in response to the same targets was also assessed using the high-affinity capture matrix assay according to manufacturer's instructions (INFγ secretion assay, Miltenyi Biotec).

### In situ tetramer staining analyses

Tissus samples were directly snap-frozen after collection and cryopreserved in liquid nitrogen until use. Serial multiple 10-µm cryostat tissue sections were used for *in situ* tetramer stainings. To block non-specific binding sites, tissue sections were first recovered with 200 µl of 1X PBS/0.5%BSA/10%FCS buffer for 15 min at room temperature. After elimination of the buffer, PE-labeled HLA-A*0201/Melan-A/Mart-1₂₆₋₃₅ or peptide-irrelevant tetramer complexes were incubated at 2.6 µg/ml concentration for 45 min at room temperature in dark humidified atmosphere. Sections were washed with PBS buffer and incubated with direct fluorescence or biotin-conjugated mAbs (anti-CD3, -CD8, -TCRVβ2, -TCRVβ3) for additionnal 30 min at 4°C. For triple staining, the biotin-coupled antibody was revealed with streptavidine-Cy5.5 conjugate. After washing, sections were fixed with PBS buffered 1% formaldehyde for 15 min at room temperature and mounted in antifading medium plus coverslip. Slides examination and micrographs were performed using a fluorescence microscope (Leica DMRXA Fluotar lenz, Weitzlar, Germany) and/or recorded using a Leica SP1 confocal microscopy system equipped with an Ar/Kr laser for 488 & 568 nm lines, and a HeNe633 laser to excite the Cy5.5 label (TCS-SP1, Leica). Simultaneous acquisition of FITC, PE and Cy5.5 labeled antibodies was performed at low illuminating laser power and with the spectrophotometer-based separation of the fluorescence emissions. The fields of interest were selected on the basis of CD8- or TCR Vβ2-FITC-labelled infiltrates to avoid bias in scoring of tetramer-PE staining prior to confocal image acquisition.

### Immunohistochemistry of infiltrating T cells and antigen-presenting cells

Slides of 4 µm in thickness were prepared from paraffin-embedded formaldehyde fixed-material, then deparaffinized, and rehydrated. For all antibodies, except for anti-CD123 and -CD68, slides were pretreated in sodium citrate at 68°C (pH=6) for 20 min. After a blocking step (Blocking agent, Ultra-tech HRP kit, Beckman-Coulter Immunotech, Marseille, France), slides were incubated with the primary antibodies at the following dilutions: 1:1 for anti-CD1a (Immunotech), 1:50 for anti-CD3 and -CD4 (Dako, Trappes, France), 1:60 for anti-p55 (Dako), 1:100 for anti-CD8 (Dako) and TIA-1 (Beckman Coulter), 1:200 for anti-Langerin (Schering-Plough), 1:300 for anti-DC Lamp and -CD123 (Schering-Plough) for 1 hour at room temperature followed by the incubation of secondary antibodies and revelation (Ultra-tech HRP kit). Slides were examined by two independent observers.

Semi-quantitative evaluation of DC-Lamp⁺ DCs infiltration was made as follow: (i) the slide was examined at intermediate magnification (x100) to select the three areas containing the maximum number of DC-Lamp⁺ DCs, (ii) the number of DC-Lamp⁺ cells per mm² was then counted at high magnification (x400) in 3 mm² per hot spot, (iii) the final counting used for DC-Lamp quantitative evaluation was the average number of the 9 countings made on the slide. Statistical analyses comparing the groups according to DC-Lamp⁺ DC infiltration utilized the Fisher's exact method and statistically significant differences were given at 95% confidence.

### EXAMPLE 1 : EVIDENCE FOR PERIPHERAL MEMORY MELAN-A/MART-1-SPECIFIC CTLS RECOGNIZING MELANOMA.

Here we report the case of a young HLA-A*0201 positive patient presenting a completely regressive primary cutaneous melanoma associated with regressing regional metastatic LNs. Postulating that this favorable clinical outcome might have resulted from an efficient anti-tumor immune response, we first examined the peripheral CTL responses at the time of the initial lymphadenectomy. The study was focused on the frequency of specific T cells recognizing the Melan-A/Mart-1 tumor antigen, one of the most immunogenic melanocytic differentiation antigen (ROMERO et al, Immunol.Rev., 188, 81-96, 2002). Direct *ex-vivo* staining of peripheral CD3⁺CD8⁺ T lymphocytes using HLA-A*0201/Mart-1₂₆₋₃₅ tetramers revealed 0.15% of specific CTLs that could be sorted by flow cytometry. Tetramer positive cells were further expanded on feeder cells either in bulk or in limiting dilution cultures. Out of 10 growing cultures, 4 tetramer⁺ CTL clones could be expanded and characterized for TCR usage and for effector functions. Immunophenotyping with TCRVβ-specific mAbs revealed two Vβ2⁺ clones (A2 and E10 utilizing BV2J2.6 and BV2J2.7 gene segments, respectively), one Vβ3⁺ clone (H3, BV3J1.5) and one Vβ13⁺ clone (C6, BV13). These CTL clones all recognized not only TAP-deficient T2 cells pulsed with Melan-A/Mart-1₂₆₋₃₅ peptide, but also the naturally processed Melan-A/Mart-1 antigen presented by the HLA-A2⁺ MEL-FON melanoma cell line, as assessed by cytotoxicity and IFNγ secretion assays. Interestingly, half-maximal lysis of peptide-pulsed T2 cells were observed with peptide concentrations ranging from 5 x10⁻⁹ to 5 x10⁻¹⁰ M, demonstrating the high TCR avidity of those clones, suggested by the bright staining with the tetramers. Such high-avidity TCR have been reported to be expressed by melanoma-reactive CTLs, while CD8⁺ T cells with impaired proliferative and effectors functions mostly display low-avidity TCR (YEE et al, J.Immunol., 162, 2227-2234, 1999).

Naive, central and effector memory T lymphocytes can be discriminated by the combined use of mAbs that allow to delineate the stages of antigenic encountering and stimulation (SALLUSTO et al, Annu.Rev.Immunol., 18, 593-620, 2000). In the peripheral blood of normal individuals, it is well established that most Melan-A/Mart-1 specific CD8⁺ T cells occur at a mean of 0.07% and are naive. In the majority of HLA-A2⁺ melanoma patients too, the majority of Melan-A/Mart-1 specific circulating CTLs occur at similar frequencies but contain variable proportions of naive and memory type cells. In marked contrast, their memory counterparts accumulate preferentially at tumor sites (ROMERO et al, 2002, mentioned above, ROMERO et al, J.Exp.Med., 188, 1641-1650, 1998). To characterize the antigenic experience status of the HLA-A*0201/Mart-1₂₆₋₃₅ tetramer⁺ CTLs found in this patient, PBMC derived-CD8⁺ T lymphocytes were analyzed by four-color immunophenotyping using anti-CD45RA, -CCR7, -TCRVβ mAbs. This confirmed the predominance of naive (CD45RA^{bright}CCR7⁺) over memory (CD45RA^{-/dim}CCR7⁻) anti-Melan-A/Mart-1 T cells. However, while the majority of tetramer⁺Vβ3⁺ CTLs appeared to be naive, most tetramer⁺Vβ2⁺ T cells displayed a memory phenotype.

### EXAMPLE 2: IN SITU CHARACTERIZATION OF TUMOR INFILTRATING MELAN-A/MART-1-SPECIFIC CTLS

The lymphocytic infiltrate of the metastatic LNs contained a majority of CD3⁺CD8⁺ lymphocytes displaying a dominant Vβ2 specificity. Importantly, a high proportion of lymphocytes were stained with the TIA-1 mAb recognizing perforin/granzyme-containing granules that are preferentially associated with memory/effector cytotoxic functions. To ascribe the Melan-A/Mart-1 specificity of tumor infiltrating CTLs, a double-staining using PE-labeled HLA-A*0201/Mart-1₂₆₋₃₅ tetramer and FITC-labeled anti-CD8 or anti-Vβ2 was performed. Significant numbers of tetramers⁺ CTLs (e.g. more than 10 cells at high magnification) were indeed infiltrating the tumor areas, about half of which were Vβ2⁺ cells. Specificity of tetramer staining was assessed using a peptide-irrelevant PE-labeled HLA-A*0201/HIVgag₇₇₋₈₅ tetramer on serial sections of the same LN and using the relevant PE-labeled HLA-A*0201/Mart-1₂₆₋₃₅ tetramer on a tumor-free alternate LN of the same axillary area. Confocal microscopy revealed in double-positive cells an overlay of FITC and PE labeling concordant with the colocalization of TCR signals obtained with tetramers and anti-TCR mAbs. Of note, tetramer staining was frequently polarized towards the T cell membrane directly in contact with adjacent tumor cells with occasional intracellular vesicles, suggesting capping and recycling of TCR containing vesicles. Those features correspond probably to dense accumulation in lipid rafts and engagement of TCR, an important process for T cell activation whose integrity is also required for efficient MHC class I tetramer binding (DRAKE and BRACIALE, J.Immunol., 166, 7009-7013, 2001).

Although direct visualization using fluorescent MHC class I/peptide complexes of antigen-specific T cells on fresh tissue sections has already been reported (SKINNER and HAASE, J.Immunol.Methods, 268, 29-34, 2002; SCHRAMA et al, J.Invest Dermatol., 119, 1443-1448, 2002), the definitive validation of this attractive technology in human tumors has not been achieved. Indeed, a major difficulty resides in the low frequency of infiltrating CTLs that are specific for a given epitope. Therefore, this assay might be applied successfully only on informative sites such as spontaneous and/or post-therapeutic tumor regressions or vitiligo areas, where it can provide convincing evidence of T cell-mediated immune responses.

Overall, Melan-A/Mart-1-specific Vβ2⁺ T lymphocytes were detected with MHC tetramer *in situ* staining of a metastatic LN draining a spontaneously regressive primary melanoma. Their close contact with tumor cells suggested their active role in the efficient natural immunosurveillance ongoing in this patient.

### EXAMPLE 3: ACCUMULATION OF MATURING DCS INTERACTING WITH TUMOR CELLS IN LN T CELL-RICH AREAS.

A panel of mAbs directed against DC surface molecules staining paraffin-embedded tissue sections was used for the the characterization of DCs infiltrating the different samples collected from this patient.

Two Langerhans cells markers, CD1a and the lectin Langerin/CD207 that are both expressed by immature LCs, were first examined.. As for the non invaded adjacent skin, numerous intra-dermal CD1a⁺ and Langerin⁺ cells were detected in the epidermis of the primary regressing cutaneous lesion, but very few in the draining metastastic LNs. Such findings contrasted with the detection of significant numbers of CD1a⁺ and Langerin⁺ cells in the subcapsular areas of normal LNs collected from other donors.

Next, in contrast to what is seen in normal LNs, no CD123/IL-3Rα⁺ DCs were observed in the LNs of the patient.

Finally, the expression of the actin-bundling protein p55/fascin (MOSIALOS et al, 1996, mentioned above) and the lysosomal protein DC-Lamp (SAINT-VIS et al, Immunity., 9, 325-336, 1998), both associated with a mature DC phenotype, was examined. In the metastatic LN as well as in the residual lymphocytic regions of the necrotic LN, a dense infiltration of p55⁺/ DC-Lamp⁺ double positive cells was observed within the T-cell rich regions and peritumoral areas, which contrasted with the scattered and mostly sub-capsular localization in a non-metastatic LN. These cells exhibited the typical morphology of "mature" DCs and were surrounded by T lymphocytes forming clusters that have been described as characteristic of ongoing immune reactions and observed at the periphery of human breast cancers (BELL et al, 1999, mentioned above). DC-Lamp⁺ or p55⁺ DCs frequently accumulated also around neomicrovessels within tumor areas. Similar observations were made in the primary skin site where p55⁺ DCs were often surrounded by lymphocytes in the regression area but not in the normal adjacent dermis, suggesting a local recruitment and/or differentiation of DC precursors from the peripheral blood. Interestingly, features of DC-Lamp staining were also different in metastatic and non metastatic LNs, with diffuse and peripheral staining of dendrites vs. concentrated and juxta-nuclear dot-like, respectively. Considering that DC-Lamp has been previously reported to accumulate in juxta-nuclear late lysosomal compartments during maturation (SAINT-VIS, 1998, mentioned above), these findings suggest that an efficient immune response is more likely associated to a maturing DC phenotype rather than a fully mature differentiation stage.

The ongoing scenario involving DC, tumor cells, T lymphocytes, and non-specific effector cells such as macrophages, orchestrated to elicit an effective *in situ* immune response remains elusive. Nonetheless, veiled dendritic p55⁺ or DC-Lamp⁺ projections tightly apposed to tumor cells were frequently observed, corroborating data from experimental *in vitro* systems in which DCs uptake tumor cell bodies and process TAA to elicit specific immune responses (BANCHEREAU et al, 2000, mentioned above). Since these DCs also interacted with several surrounding lymphocytes, one can postulate that such interactions may trigger *in situ* activation, expansion and effectors functions of antigen-specific T cells culminating, like in this patient, in tumor regression.

### EXAMPLE 4: THE DENSITY OF DC-LAMP⁺ DCS IN MELANOMA-POSITIVE SENTINEL LYMPH NODE IS INVERSELY CORRELATED WITH METASTASIS IN DOWNSTREAM LYMH NODES.

Since the most striking observation associated with the spontaneous tumor regression was the dramatic accumulation of DC-Lamp⁺ mature DCs in the metastatic LN, the presence of mature DCs was analyzed in a series of 19 melanoma-positive SLNs. Since SLNs are recognized as the first site of melanoma metastases, it was postulated that the extent of mature DC infiltrates may dictate the induction of efficient local anti-tumor immune responses. Therefore, the following parameters were evaluated by two independent observers: i) a semi-quantitative evaluation of the absolute number of DCs counted at sites of highest SLN infiltration that led to the identification of two groups of patients: SLN/DC-Lamp^{low} (+ or ++, i.e. <100 or 100-300 DC-Lamp⁺/mm², n=11) and SLN/DC-Lamp^{high} (+++; i.e.>300 DC-Lamp⁺/mm², n=8); ii) the aspect of DC-Lamp staining, i.e. dendritic or dot-like, that may correspond to a maturing or fully mature phenotype, respectively; iii) when tumor cells could be visualized (in only 10 out of the 19 SLN), tumor infiltration of DC-Lamp⁺ cells and evidence for clusters of DC-Lamp⁺ cells with tumor cells and lymphocytes. Results are summarized in Table 1. In the SLN/DC-Lamp^{low} group, DC-Lamp⁺ cells displayed a predominant dot-like lysosomal staining whereas in the SLN/DC-Lamp^{high} group, DC-lamp⁺ cells displayed a predominant dendritic staining (p=0.019) and an increased frequency of tumor infiltration and DC clustering, as mentioned in the case report. Furthermore, there was a striking correlation between the density of DC-Lamp infiltrates and the occurrence of at least one melanoma-positive non-SLN in the subsequent regional completion lymphadenectomy performed few weeks after the SLN resection. Seven out of 11 patients of the SLN/DC-Lamp^{low} group had at least one metastatic non-SLN, in contrast to none out of 8 patients of the SLN/DC-Lamp^{high} group (p=0.013).

**TABLE I**

| **Parameters analyzed** | **SLN/DC-Lamp**^{**low**} | **SLN/DC-Lamp**^{**high**} | **Significance**^{**a**} |
|---|---|---|---|
| **Density (DC-Lamp**^{**+**}**cells/mm**^{**2**}**)** | < 300 | > 300 | - |
| **Patients (n)** | 11 | 8 | - |

| **Morphology of DC-Lamp Staining (n)** | | | |
|---|---|---|---|
| > Dendritic | 3 | 7 | |
| > Dot-like | 8 | 1 | p=0.0198 |

| **Infiltration of Tumor by DC-Lamp**^{**+**} **cells**^{**b**} | | | |
|---|---|---|---|
| > Yes | 2/6 | 3/4 | ns |
| > Clusters DCs/tumor cells/lymphocyte | 1/6 | 2/4 | ns |
| > No | 4/6 | 1/4 | ns |
| **Patients with Metastatic non-SLNsin Lymphadenectomy (n)** | 7 | 0 | p=0.0128 |

| | | | |
|---|---|---|---|
| ^{a} using the Fischer's exact test. | | | |
| ^{b} Tumor areas evidenced on the slides of 10 positive SLNs used for DC-Lamp immunohistochemistry | | | |

## Claims

1. A method for evaluating the risk of metastatic dissemination of a tumor in a patient wherein said method comprises determining the number of dendritic cells in a sample taken from sentinel lymph nodes of said patient.

2. A method for evaluating the clinical efficiency of anti-tumor immunotherapy in a patient wherein said method comprises determining the number of dendritic cells in a sample taken from sentinel lymph nodes of said patient.

3. The method of any of claims 1 or 2 wherein said dendritic cells are DC-Lamp+ dendritic cells

4. The method of any of claims 1 to 3 wherein said tumor is a melanoma.
